# EUROPEAN PATENT APPLICATION

(11) **EP 4 674 579 A1**
(43) Date of publication of application: **07.01.2026**
(21) Application number: 24306125.6
(22) Date of filing: 05.07.2024
(51) Int. Cl.: B25J 15/04, A61B 34/30, A61B 17/00, A61B 90/00

(54) **CONNECTING PIECE FOR CONNECTING AN END-EFFECTOR WITH A ROBOTIC ARM**

(71) Applicant: MinMaxMedical, 38400 Saint-Martin-d'Hères (FR)
(72) Inventor: FOURNIER, Elie, 38400 Saint-Martin-d'Hères (FR); BEAUSSE, Cédric, 38400 Saint-Martin-d'Hères (FR); COLLET, Hervé, 38400 Saint-Martin-d'Hères (FR)
(74) Representative: Regimbeau

(57) **Abstract**

The present invention concerns a connecting piece (200) for connecting an end-effector with a robotic arm, the connecting piece (200) comprising at least a first part (210) configured to detachably engage with a second part (220), one of the first part (210) and the second part (220) being attached to the robotic arm and the other of the first part (210) and the second part (220) being attached to the end-effector, the first part (210) comprising a female coupling element and the second part (220) comprising a male coupling element, the connecting piece (200) being configured to connect electrically and mechanically the end-effector to the robotic arm, wherein the first part (210) comprises a retractable device (230) configured to shutter the female coupling element when the first part (210) and the second part (220) are disengaged.

## Description

The present invention pertains to the general domain of surgical systems, and more particularly to surgical systems with interchangeable end-effectors.

During the last few decades, the field of surgical systems has significantly grown, and especially surgical systems which are adapted to be used in computer-assisted medical intervention (CAMI), as referred to in the publication of S. Lavallée & P. Cinquin: "Computer assisted medical interventions" In K.H. Hohne, editor, NATO ARW, 30 Imaging in Medicine, Vol F60, 301-312, Berlin, June 1990. Springer-Verlag. Such systems aim to help surgeons in performing securer surgeries while also improving the accuracy and reproducibility of said surgeries. The use of those surgical systems also aims at improving the precision of surgical movements, lower invasiveness and at reducing the number and the seriousness of errors that can otherwise occur during those surgeries.

Robotic aid to clinicians for execution of optimal surgery was introduced in the early 1980's in neurosurgery application. Since the 1990's, several assistive technologies combining part or all of 2D/3D imaging, navigation and robotics were developed with the primary focus of improving accuracy of surgical procedures, in view of improving clinical and functional outcomes for the patients.

A first generation of robots were developed as passive-robots. These passive-robots can for example consist of optical localizer or motorless encoded arms and they are particularly well-suited for navigation, but their use is difficult for executing complex surgical strategies. However, these passive-robots can be useful for performing simple operation such as ones wherein the movements needed are all about one single axis. For instance, the robot Cirq^{®} developed by BrainLab is one of those passive robots.

A second generation of robots were developed as active robots. These active robots are designed to perform at least part of an intervention on their own from a planned procedure, i.e., without any real-time guidance from the surgeon, nor from any other operator. For instance, Robodoc^{®} is an active robot developed by Integrated Surgical Systems, commercialized at the end of the 90's and adapted to perform part of hip replacements surgeries. Such active robots are generally accurate but raise safety and ergonomics issues.

Nowadays, many medical device suppliers are developing collaborative robots, i.e., robots with which the operators are able to cooperate. These collaborative robots are equipped with an input device through which the user of the system can ask for a displacement of the robot, and the system is then adapted to treat the received input and to displace the robot literally as asked by the user or to forbid the displacement asked, i.e. taking into account both the demand of the user and, for instance, safety parameter. One major drawback of those collaborative robots is that they are particularly adapted to be used for one dedicated kind of surgery, thus forcing the users to buy several robots if they want to be able to perform different surgeries.

Thus, there remains a need to provide a collaborative surgical system which can be used for several kind of surgeries.

The present invention falls in this context and aims at providing a connecting piece for connecting an end-effector with a robotic arm allowing a quick and easy change of the end-effector, thus allowing to use a unique robotic arm to perform different kind of surgeries.

An object of the invention thus concerns a connecting piece for connecting an end-effector with a robotic arm, the connecting piece comprising at least a first part configured to detachably engage with a second part, one of the first part and the second part being attached to the robotic arm and the other of the first part and the second part being attached to the end-effector, the first part comprising a female coupling element and the second part comprising a male coupling element, the connecting piece being configured to connect electrically and mechanically the end-effector to the robotic arm, wherein the first part comprises a retractable device configured to shutter the female coupling element when the first part and the second part are disengaged.

Thus, the female coupling element is protected by the retractable device when the first part and the second part are disengaged. Advantageously, the connecting piece can therefore be easily cleaned, as such retractable device flatten the surface to be cleaned.

The invention can further be completed by one or several of the following features taken alone or in combination.

One of the first part and the second part houses a plurality of electrical contacts configured for electrically connecting the end-effector with the robotic arm. For instance, the first part and the second part present circular shapes and the plurality of electrical contacts is housed in a center of the first part or of the second part of the connecting piece. The connecting piece comprises at least two electrical contacts. Advantageously, the connecting piece comprises at least 30 electrical contacts.

The connecting piece comprises at least one elastic element configured to urge the retractable device in a shuttering position when the first part and the second part are disengaged. By "shuttering position", we here refer to a position in which the retractable element shutters the female coupling element, thus making the surface flat and easy to clean.

The elastic element can be a unique circular elastic element.

The elastic element can comprise a plurality of elastic elements.

The female coupling element and the male coupling element form a bayonet-type connection between the first part and the second part.

The connecting piece comprises a locking device configured to prevent disengagement of the first part with respect to the second part.

The locking device can comprise a locking ring configured to cooperate with at least one locking pin. The locking ring can be configured to be rotated along only one locking direction. The locking device can be configured to prevent rotation of the locking ring along an unlocking position.

The connecting piece comprises a safety device configured to generate at least one signal when the female coupling element and the male coupling element are engaged with each other.

The safety device comprises a first component arranged on one of the first part and the second part and a second component arranged on the other of the first part and the second part, the first component and the second component being arranged so as to face each other when the male coupling element and the female coupling element are engaged with each other.

The first component of the safety device is a magnet and wherein the second component of the safety device is a Hall effect sensor.

The safety device comprises a manual switch connected in series with the electrical contacts or as a separate signal.

The connecting piece presents a cylindrical shape extending mainly along a longitudinal axis. The retractable device is mobile along the longitudinal axis between the shuttering position and a retracted position.

An object of the present invention further concerns a surgical system comprising at least a data processor, a robotic arm, and an end-effector comprising a surgical instrument and an input device, the end-effector being configured to be connected to the robotic arm by a connecting piece according to the present disclosure, the data processor being configured to receive commands, at least, from the input device and to compute instructions to displace the robotic arm so as to implement the received commands. The connecting piece comprises a safety device configured to send the signal generated when the female coupling element and the male coupling element are engaged with each other to the data processor and wherein the data processor is configured to execute the computed instruction(s) only when the signal is generated by the safety device. Thus, the input device is arranged after the connecting piece, i.e., between the connecting piece and the surgical instrument of the end-effector.

Further details and advantages will be described with more details below with reference to the accompanying drawings.
Figures 1 and 2 illustrate part of a surgical system 100 according to, respectively, a first configuration and a second configuration;
Figure 3 is a perspective view of a connecting piece according to an embodiment of the invention;
Figures 4 and 5 are two perspective views of a first part of the connecting piece illustrated on figure 3, figure 4 illustrating a first conformation of the first part and figure 5 illustrating a second conformation of this first part;
Figure 6 is a perspective view of a second part of the connecting piece illustrated on figure 3;
Figure 7 is a cross-section view of the connecting piece illustrated on figure 3, the cross-section being realized according to the plane A-A illustrated on said figure 7A which is a top view of the connecting piece;
Figure 8 is a perspective view of a locking ring of the connecting piece;
Figures 9A to 9D are close-up views of part of the connecting piece illustrating the locking of the first part with respect to the second part of the connecting piece, via a locking device of such connecting piece;
Figures 10A and 10B illustrate a safety device of the connecting piece, represented respectively in an open position and in a close position.

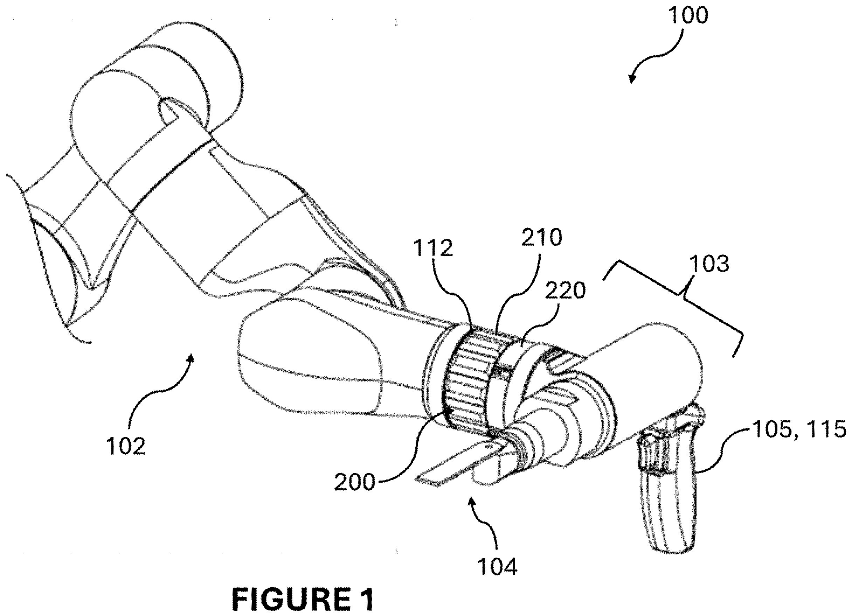

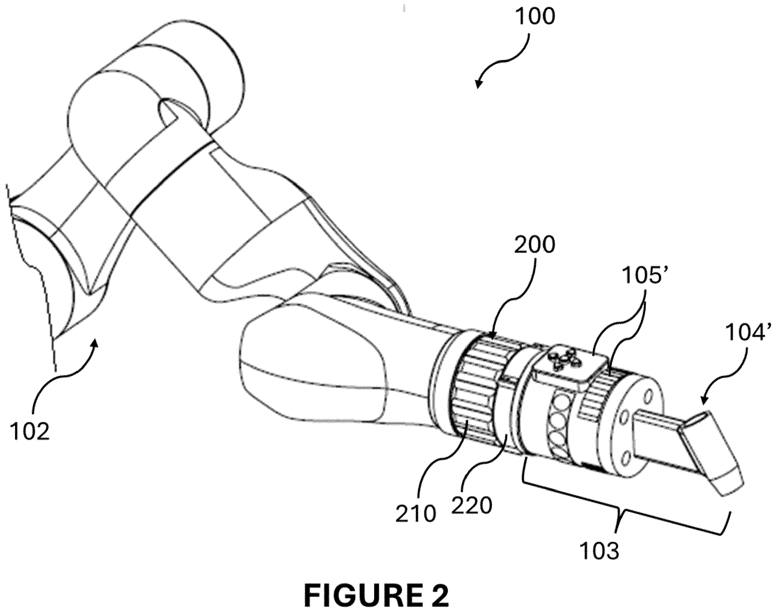

Figures 1 and 2 illustrate part of a surgical system 100 according to, respectively, a first configuration and a second configuration. In both configurations, the surgical system 100 comprises a cart - not shown - from which extends a robotic arm 102. The robotic arm 102 extends from the cart to a flange 112. An end-effector 103 is connected to the flange 112 thanks to a connecting piece 200. The end-effector 103 comprises a surgical instrument 104, 104' and an input device 105, 105'. The surgical system 100 further comprises at least one data processor - for instance housed in the cart - configured to receive commands from, at least, the input device 105, 105' and to compute instructions to displace the robotic arm 102 in order to implement the received commands. As illustrated, the input device 105, 105' is arranged after the connecting piece 200, i.e., between the connecting piece 200 and the surgical instrument 104, 104' of the end-effector 103.

The first configuration and the second configuration differ from one another in the features of the end-effector. According to the first configuration illustrated on figure 1, the surgical instrument 104 is an active instrument, meaning that the surgical instrument is adapted to be used to machine the anatomical structure to be treated while according to the second configuration illustrated on figure 2, the surgical instrument 104' is a passive instrument, here a guide configured to guide a surgical tool - not illustrated - held by a surgeon, user of the system. The input device 105, 105' also differs between the first and the second configurations. The input device 105 according to the first configuration for instance comprises a handle 115 linked to a force sensor. The input device 105' according to the second configuration for instance comprises buttons 115' arranged on the end-effector 103. The connecting piece 200 however is identical between the two configurations. Such connecting piece thus permits to connect different kinds of end-effectors 103 to a unique kind of robotic arm 102, thus advantageously allowing to perform several distinct surgeries with a unique surgical system 100. The differences between the two configurations will therefore not be described with more details in this document.

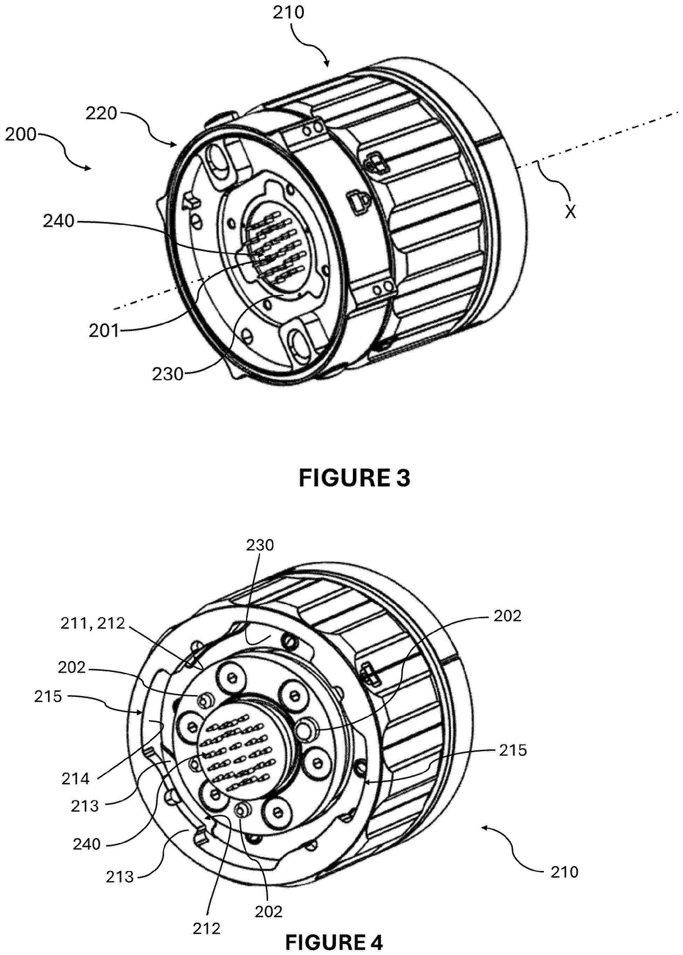

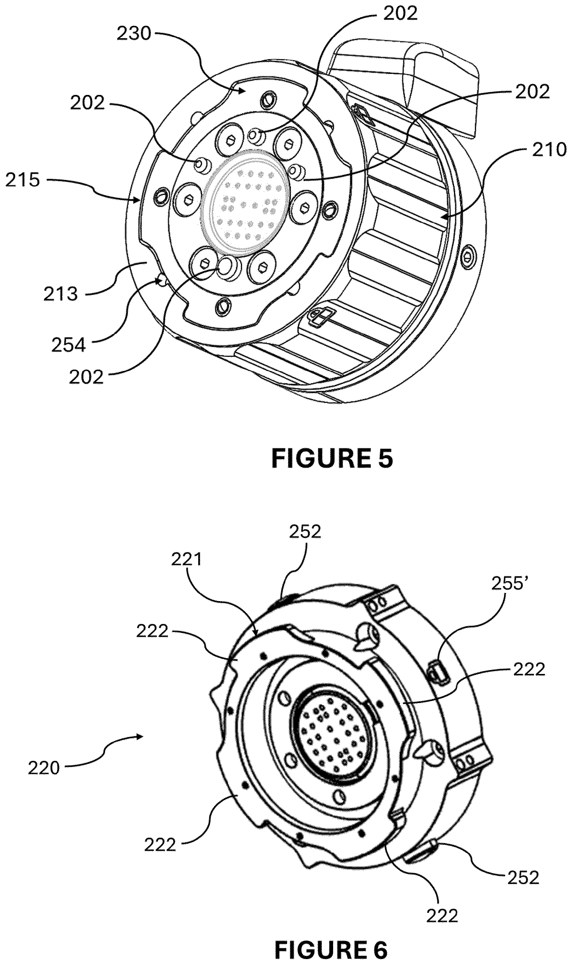

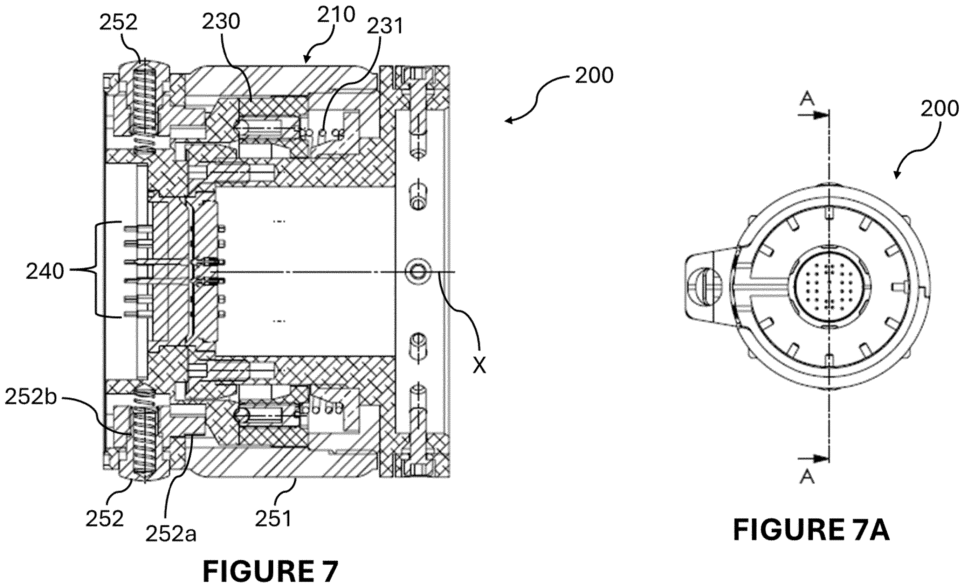

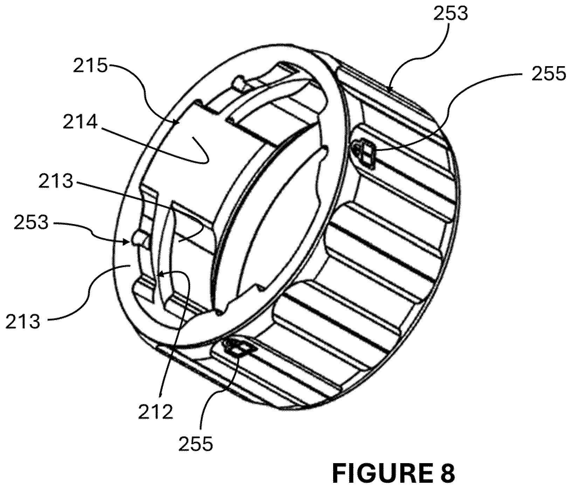

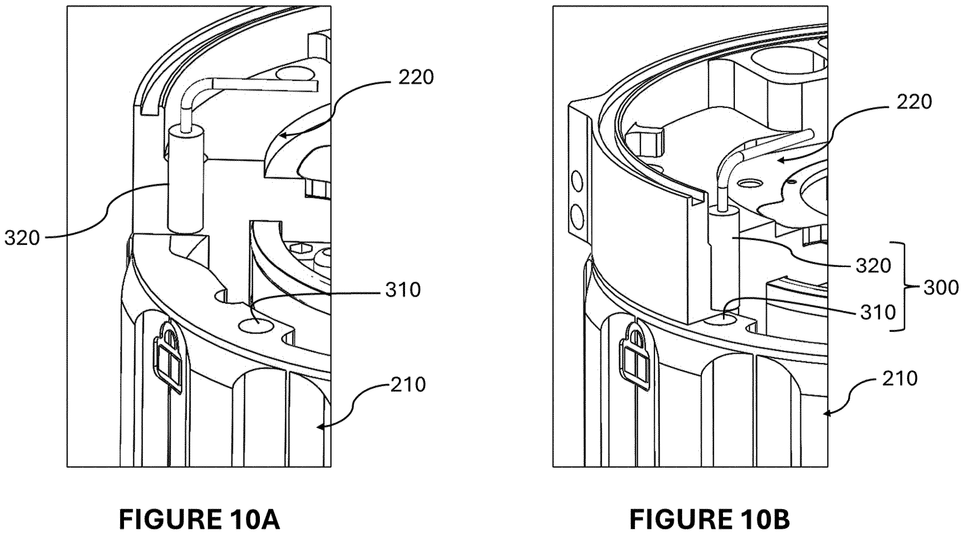

Now referring to figures 3 to 10, the connecting piece 200 will be detailed. Figure 3 is a perspective view of the connecting piece 200. Figure 4 and figure 5 illustrate a perspective view of a first part 210 of the connecting piece 200 according, respectively, to a first conformation and to a second conformation. Figure 6 is a perspective view of a second part 220 of the connecting piece 200. Figure 7 is a cross-section view of the connecting piece 200 taken along plane AA illustrated on figure 7A which is a top view of the connecting piece 200. Figure 7A thus makes visible a cooperation between the first part 210 and the second part 220 of the connecting piece 200. Figures 8 to 9D illustrate, according to different views, a locking device of the connecting piece 200. Finally, figures 10A and 10B illustrate a safety device of the connecting piece 200, represented according to, respectively, an open position and a close position.

The connecting piece 200 comprises a first part 210 configured to detachably engaged with a second part 220. According to the illustrated embodiment, the connecting piece 200 presents a cylindrical shape extending mainly along a longitudinal axis X. One of the first part 210 and the second part 220 is attached to the robotic arm while the other of the first part 210 and the second part 220 is attached to the end-effector 103. According to the embodiment illustrated on figures 1 and 2, the first part 210 is attached to the robotic arm 102, and especially to the flange of the robotic arm 102, while the second part 220 is attached to the end-effector 103. It is understood that the positions of these parts 210, 220 could be inverted without departing from the scope of the invention.

The first part 210 and the second part 220 are coupled to one another thanks to a male-female connection. The first part 210 thus comprises a female coupling element211 and the second part 220 comprises a male coupling element 221. According to the illustrated embodiment, the female coupling element 211 and the male coupling element 221 form a bayonet-type connection between the first part 210 and the second part 220. Thus, the first part 210 is configured to be rotated with respect to the second part 220 so as for the male-female connection to engage. Especially, the female coupling element 211 comprises at least one groove 212 and the male coupling element 221 comprises at least one wing 222 configured to be received in the corresponding groove 212. According to the embodiment shown, the female coupling element 211 comprises four grooves 212 - only two being referenced on figure 4 - and the male coupling element 221 comprises four corresponding wings 222. Particularly, each groove 212 is formed between two parallel flanges 213 extending from an internal face 214 of the first part 210.

The wings 222 thus form a ramp configured to engage the grooves. In other words, the wings 222 form a ramp configured to slide into the grooves as the first part is rotated with respect to the second part. This cooperation is for instance illustrated on figure 7.

The connecting piece 200 is configured to connect electrically and mechanically the end-effector to the robotic arm. To implement the electrical connection between the end-effector and the robotic arm one of the first part 210 and the second part 220 houses at least two electrical contacts 240 configured for electrically connecting the end-effector with the robotic arm. According to the illustrated embodiment, the first part 210 and the second part 220 present circular shapes and the electrical contacts 240 are housed in a center 201 of the connecting piece 200. For instance, the electrical contacts can be spring-loaded contacts. The first part 210 further comprises a retractable device 230 configured to shutter the female coupling element 211 when the first part 210 and the second part 220 are disengaged. Such retractable device is for instance illustrated on figures 4 and 5. This retractable device 230 is retracted when the first part 210 and the second part 220 are engaged - as for instance illustrated on figure 4 - and deploys when the first part 210 and the second part 220 are disengaged - as for instance illustrated on figure 5. Figure 4 thus illustrates a first conformation of the first part 210 wherein the retractable device 230 is in a retracted position while figure 5 illustrates a second conformation of the first part 210 wherein the retractable device 230 is in a shuttering position. The retractable device 230 ensures that the female coupling element 211 is protected when the first part 210 and the second part 220 are disengaged. The retractable device 230 is thus mobile along the longitudinal axis X between the retracted position and the shuttering position. Advantageously, this retractable device 230 facilitates the cleaning of the connecting piece 200, as said retractable device 230 flattens the surface to be cleaned, as illustrated on figure 5.

As shown, the retractable device 230 has a general circular shape with a central opening. The retractable device 230 is configured to fill spaces 215 formed between the flanges 213 described above, thus making the surface flat and easy to clean as mentioned. In other words, when the retractable device 230 is in the shuttering position, a superior face of the retractable device 230 and a superior face of the first part 210 both extend in a common plane.

Furthermore, the connecting piece 200 comprises at least one keying pin 202 ensuring that the electrical contacts 240 are connected to the flange according to a predefined orientation. According to the illustrated embodiment, the connecting piece 200 especially comprises four keying pins 202. Obviously, at least one of the four keying pins 202 must be different from the three other ones to avoid a symmetrical arrangement of said keying pins 202. According to the illustrated embodiment, one of the keying pins 202 is thus bigger than the three other ones. Advantageously, these keying pins 202 further facilitate the assembly of the connecting piece 200 on the flange.

As illustrated on figure 7, the connecting piece 200 comprises at least one elastic element 231 configured to urge the retractable device 230 in the shuttering position when the first part 210 and the second part 220 are disengaged. Figure 7 illustrates the second conformation of the connecting piece 200, i.e., the conformation wherein the first part 210 and the second part 220 are engaged with each other. The elastic element 231 is thus illustrated in a compressed state on figure 7. For instance, the elastic element 231 is a unique circular elastic element. Alternatively, the elastic element 231 comprises a plurality of springs.

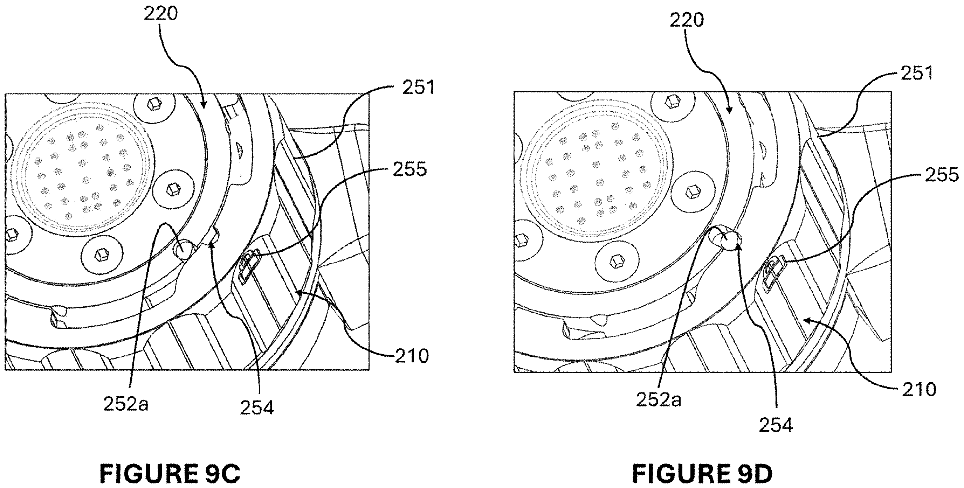

The connecting piece 200 further comprises a locking device 250 configured to prevent disengagement of the first part 210 with respect to the second part 220. According to the illustrated example, this locking device 250 is more particularly distributed between the first part and the second part 220. The locking device 250 can for instance comprise a locking ring 251 illustrated, in a perspective view, on figure 8 and at least one push button 252 configured to engage with the locking ring 251, as for instance shown on figures 7 and 9D. As shown, the locking ring 251 presents a general cylindrical shape with an internal face 214 which also forms the internal face of the female coupling element 211 and an external face 253. At least one notch 254 - advantageously four notches 254 as shown on the illustrated embodiment - is formed on one of the flanges 213 extending from the internal face 214, as described above.

The at least one push button 252 comprises a locking pin 252a and a spring 252b configured to urge the locking pin 252a in a locking position, i.e., toward the internal face 214 of the locking ring 251 from which extend the flanges 213 encompassing the notches 254. The spring 252b is for instance illustrated on figure 7.

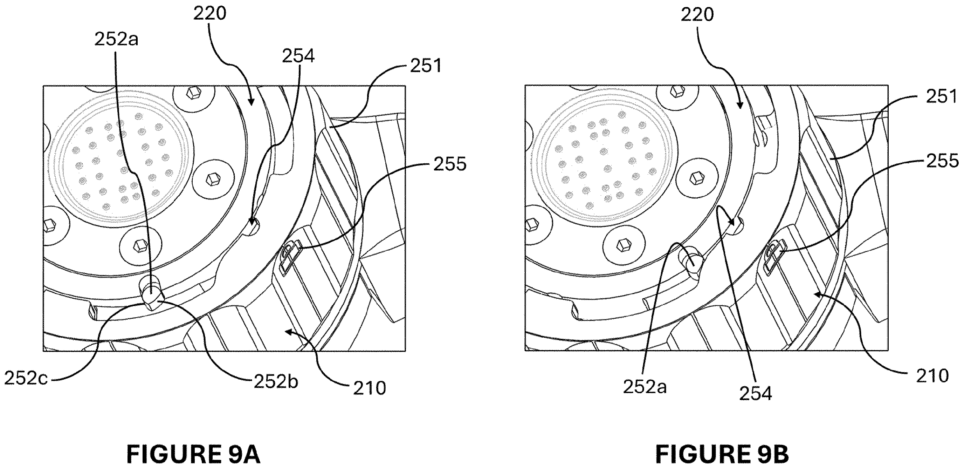

Additionally, the locking pin 252a comprises a first face 252b and a second face 252c with different shapes. Particularly, the shape of the first face 252b is configured to allow rotation of the locking ring 251 in one direction, while the shape of the second face 252c is configured to block and prevent rotation - and thus disengagement - of the locking ring 251 in the opposite direction once that the locking pin 252a is engaged into the notch 254. Furthermore, the first face 252b is configured to allow retraction of the locking pin 252a toward the center of the connecting piece, thus allowing rotation of the locking ring 251 from an open position - for instance illustrated on figures 9a, 9B, 9C and 10A - to a close position - for instance illustrated on figures 9D and 10B.

According to a particular embodiment, the first face 252b of the locking pin 252a is a side slope 252b and the second face 252c is a straight side 252c, thus ensuring that the locking ring 251 can only be rotated according to one unique direction, the straight side 252c forming a hard stop to the rotation along the opposite direction.

Figures 9A to 9B illustrate successive steps to lock the position of the first part 210 with respect to the second part 220 of the connecting piece 200. As illustrated, as the locking ring 251 is rotated counterclockwise as seen from the second part 220, the flanges 213 come into contact with the locking pins 252a, and particularly with the side slopes 252b of such locking pins 252a, thus pushing the locking pins 252a toward the center of the connecting piece. Once the locking ring 251 is sufficiently turned - here about a quarter turn-, the locking pins 252a engage the corresponding notches 254, thus locking the position of the first part 210 with respect to the second part 220 of the connecting piece 200. At least one visual indicator 255 is also engraved in the external face 253 of the locking ring 251. Particularly, each visual indicator 255 is engraved at a position where a notch 254 is formed. At least one corresponding visual indicator 255' is engraved on the second part 220, at a position where the push button 252 is arranged. Thus, the user can use the visual indicators 255, 255' of the first part 210 and of the second part 220 to ensure that the push buttons 252 face the corresponding notches 254.

It is understood from figure 7 that pressing the push buttons 252 so as to compress the spring 252b results in a displacement of the locking pins 252a, permitting disengaged the locking pins 252a from the notches 254 and thus to rotate the locking ring 251. The first part 210 and the second part can then be disengaged from each other, and the end-effector can thus be disconnected from the flange.

Moreover, the connecting piece 200 comprises a safety device 300 configured to generate a signal when the female coupling element and the male coupling element are engaged with each other. For instance, the safety device 300 comprises a first component, for instance a magnet 310, arranged on one of the first part 210 and the second part 220 and a second component, for instance a Hall effect sensor 320 arranged on the other of the first part 210 and the second part 220. Particularly, the first component and the second component are here arranged so that they face each other when the two parts of the locking device - here formed as the push buttons and the locking ring - are engaged with each other. As illustrated on figures 10A and 10B, the magnet 310 is advantageously arranged on the locking ring 251 which forms a mobile part of the locking device. This safety device 300 thus ensures that the first part 210 and the second part 220 of the connecting piece 200 are mechanically engaged with each other.

Additionally, the connecting piece 200 can be configured to ensure that the electrical connection is correctly realized to the flange of the robotic arm. To ensure that the electrical contacts are plugged to the flange, a current can be applied on a series circuit comprising said electrical contacts. If the current is measured, the power can be turned on. Alternatively, the safety device can comprise a manual switch connected in series with the electrical contacts or as a separate signal.

As mentioned above, the data processor of the surgical system is configured to receive commands, at least, from the input device and to compute instructions to displace the robotic arm so as to implement the received commands. Advantageously, the data processor is further configured to execute the computed instruction(s) only when the signal is generated by the safety device.

## Claims

1. A connecting piece (200) for connecting an end-effector (103) with a robotic arm (102), the connecting piece (200) comprising at least a first part (210) configured to detachably engage with a second part (220), one of the first part (210) and the second part (220) being attached to the robotic arm (102) and the other of the first part (210) and the second part (220) being attached to the end-effector (103), the first part (210) comprising a female coupling element (211) and the second part (220) comprising a male coupling element (221), the connecting piece (200) being configured to connect electrically and mechanically the end-effector (103) to the robotic arm (102), wherein the first part (210) comprises a retractable device (230) configured to shutter the female coupling element (211) when the first part (210) and the second part (220) are disengaged.

2. The connecting piece (200) according to the preceding claim, wherein one of the first part (210) and the second part (220) houses a plurality of electrical contacts (240) configured for electrically connecting the end-effector (103) with the robotic arm (102).

3. The connecting piece (200) according to any of the preceding claims, comprising at least one elastic element (231) configured to urge the retractable device (230) in a shuttering position when the first part (210) and the second part (220) are disengaged.

4. The connecting piece (200) according to the preceding claim, wherein the elastic element (231) is a unique circular elastic element (231).

5. The connecting piece (200) according to claim 3, wherein the elastic element (231) comprises a plurality of elastic elements.

6. The connecting piece (200) according to any of the preceding claims, wherein the female coupling element (211) and the male coupling element (221) form a bayonet-type connection between the first part (210) and the second part (220).

7. The connecting piece (200) according to any of the preceding claims, comprising a locking device (250) configured to prevent disengagement of the first part (210) with respect to the second part (220).

8. The connecting piece (200) according to any of the preceding claims, comprising a safety device (300) configured to generate at least one signal when the female coupling element (211) and the male coupling element (221) are engaged with each other.

9. The connecting piece (200) according to the preceding claim, wherein the safety device (300) comprises a first component arranged on one of the first part (210) and the second part (220) and a second component arranged on the other of the first part (210) and the second part (220), the first component and the second component being arranged so as to face each other when the male coupling element (221) and the female coupling element (211) are engaged with each other.

10. The connecting piece (200) according to the preceding claim, wherein the first component of the safety device (300) is a magnet (310) and wherein the second component of the safety device (300) is a Hall effect sensor (320).

11. The connecting piece (200) according to any of claims 8 to 10, wherein the safety device (300) comprises a manual switch connected in series with the electrical contacts (240) or as a separate signal.

12. The connecting piece (200) according to any of the preceding claims, presenting a cylindrical shape extending mainly along a longitudinal axis (X).

13. The connecting piece (200) according to the preceding claim, wherein the retractable device (230) is mobile along the longitudinal axis (X) between a shuttering position and a retracted position.

14. Surgical system comprising at least a data processor, a robotic arm (102), and an end-effector (103) comprising a surgical instrument (104, 104') and an input device (105, 105'), the end-effector (103) being configured to be connected to the robotic arm (102) by a connecting piece (200) according to any of the preceding claims, the data processor being configured to receive commands, at least, from the input device (105, 105') and to compute instructions to displace the robotic arm (102) so as to implement the received commands.

15. Surgical system according to the preceding claim, wherein the connecting piece (200) comprises a safety device (300) configured to send the signal generated when the female coupling element (211) and the male coupling element (221) are engaged with each other to the data processor and wherein the data processor is configured to execute the computed instruction(s) only when the signal is generated by the safety device (300).
